# FASCICULE DE BREVET EUROPEEN

(11) **EP 0 983 755 B1**
(45) Date de publication et mention de la délivrance du brevet: **28.05.2008**
(21) Numéro de dépôt: 99420186.1
(22) Date de dépôt: 06.09.1999
(51) Int. Cl.: A61F 2/42

(54) **Implant pour pied plat**
Plattfussimplantat
Implant for flat foot

(30) Priorité: 04.09.1998 FR 9811107
(43) Date de publication de la demande: 08.03.2000
(73) Titulaire: European Foot Platform, 54000 Nancy (FR)
(72) Inventeur: Viladot Perice, Ramon, 08022 Barcelona (ES); Dereymaeker, Greta, 3030 Oud-Heverlee (BE); Diebold, Patrice Francois, 54000 Nancy (FR); Hintermann, Beat, 4125 Riehen (CH)
(74) Mandataire: Martin, Didier Roland Valéry

(56) Documents cités:
- EP-A- 0 560 249
- WO-A-97/29693
- FR-A- 2 543 821
- FR-A- 2 653 660
- US-A- 4 450 591

## Description

La présente invention concerne un dispositif d'implant pour pied plat, et plus particulièrement un dispositif d'implant expansif pour reformer la voûte plantaire naturelle dans la pathologie du pied plat.

Pour remédier au pied plat valgus de l'enfant, qui est dû à la perte des rapports anatomiques normaux de l'astragale et du calcanéum, on a eu récemment l'idée d'introduire dans le sinus du tarse, en matière de cale, du matériel prothétique. Cependant, cette introduction d'un tel matériel prothétique pose le problème du maintien du matériel dans le sinus tarsien, qui anatomiquement est un entonnoir ouvert en dehors. Ainsi, on a implanté une vis pour arthrodèse astragalo-calcanéenne temporaire, restituant aux différentes pièces leurs rapports anatomiques normaux, qui fait agir l'action remodelante de la croissance de l'enfant. La croissance gomme la dysplasie et restitue la cohésion de l'ensemble. Le maintien est réalisé par une vis trans-astragalo-calcanéenne et, une fois le remodelage réalisé, la fixation temporaire est démontée, libérant le couple sous-astragalien.

Dans les années 1975, on a utilisé une prothèse en silicone de forme générale tronconique, et qui possède un appendice. L'ensemble est très proche de la forme d'une flûte de champagne. Sa géométrie lui permet d'être très congruente au logement dans lequel il doit être implanté. Ainsi, sa forme permet à cette prothèse de s'adapter parfaitement dans le logement. Les inconvénients d'une telle prothèse sont liés, d'une part, à la matière de silicone dont le comportement face à une sollicitation mécanique n'est pas totalement connu et, d'autre part, à sa technique opératoire. En effet, pour introduire cet implant, le praticien doit réaliser une double incision, l'une sur la face interne du pied et l'autre sur la face externe.

Pour pallier ces inconvénients, on a réalisé un autre dispositif, qui peut être implanté en ne réalisant qu'une seule incision. Cette endo-orthèse est fabriquée en polyéthylène haute densité, et a la forme d'un cylindre comportant une jante à sa base. Elle est percée en son centre d'un tunnel dans lequel on introduit une vis. Elle est découpée dans ses deux tiers antérieurs en quatre quartiers, destinés à s'expanser sous l'effet de l'avancée de la vis dans son axe. L'endo-orthèse est filetée intérieurement. Ce dispositif a pour fonction d'ouvrir le canal du sinus tarsien pour repositionner l'astragale sur le calcanéum. L'inconvénient majeur de cet implant est que sa forme expansée n'est absolument pas congruente à la cavité du sinus tarsien, même après serrage de la vis interne dans le tunnel.

Un dispositif analogue, mais fabriqué en matériau biodégradable, est décrit dans le document EP-A-560249.

L'invention vise donc à remédier à ces inconvénients.

Un but de l'invention est de fournir un implant pour pied plat qui soit expansif et qui ne nécessite q'une seule incision.

Un autre but de l'invention est de fournir un implant pour le traitement de pied plat qui soit congruent à la cavité du sinus tarsien.

Un autre but de l'invention est encore de fournir un implant pour pied plat dont l'expansion soit répartie de façon homogène diamétralement.

Un quatrième but de l'invention est de fournir un implant pour pied plat qui puisse être correctement logé dans la cavité du sinus tarsien, en étant bloqué facilement.

L'invention concerne donc un dispositif chirurgical pour pied plat, caractérisé en ce qu'il comprend trois parties :
- un corps central cylindrique comportant un filetage sur sa partie externe, comportant une tête de blocage en translation,
- un cône d'expansion comportant d'une part un filetage à sa partie proximale (partie la plus interne lorsque le dispositif est implanté), coopérant avec le filetage du corps central,
- une couronne externe pouvant être expansée par le cône d'expansion, lors du vissage du corps central qui translate longitudinalement le cône d'expansion dans la couronne externe, depuis la partie distale vers la partie proximale.

Le dispositif chirurgical selon la présente invention est avantageusement encore tel que :

La couronne externe est constituée d'un matériau déformable biocompatible et /ou biodégradable, par exemple du polyéthylène ou un métal.

Le corps central est avantageusement métallique, par exemple en titane ou en acier inoxydable.

Le cône d'expansion est avantageusement métallique, par exemple en titane ou en acier inoxydable. Chacune de ces trois parties peut néanmoins être réalisée en tout matériau biocompatible susceptible d'avoir les propriétés mécaniques requises, et notamment de déformabilité dans le cas du cylindre extérieur et de résistance mécanique dans le cas du corps central et du cône d'expansion.

Le cône d'expansion, lors du vissage du corps central, expanse la couronne externe d'une valeur comprise entre 0,5 mm et 2 mm sur toute la longueur, et de manière homogène diamétralement.

La couronne externe possède des entailles sur sa surface externe et des entailles sur sa surface interne, d'une profondeur supérieure à la moitié de l'épaisseur de la couronne, et décalées angulairement, de manière à ce que la couronne externe soit expansée en augmentant son diamètre.

La couronne externe possède des moyens anti-recul, notamment des ailettes, dont l'angle médian a une valeur comprise entre 80° et 20° par rapport à l'axe longitudinal du dispositif, de préférence de 45°, et ont une largeur comprise entre 1 mm et 2 mm, de manière à permettre un meilleur blocage du dispositif dans la cavité du sinus tarsien.

Le corps central possède à sa partie distale un usinage permettant le montage d'un instrument pour l'entraîner en rotation.

Le cône d'expansion a une face externe lisse.

Le cône d'expansion a un filetage à gauche dans sa partie proximale et un usinage dans sa partie distale, notamment un filetage à droite, de manière à permettre de monter un préhenseur.

Le cône d'expansion comporte un second usinage, notamment une fente, pour permettre l'introduction d'un autre instrument, de manière à bloquer en rotation le dispositif, et pour permettre sa translation le long de la couronne externe pendant la rotation du corps central.

La description suivante, en regard des dessins annexés à titre d'exemples non limitatifs, permettra de mieux comprendre comment l'invention peut être mise en pratique.
- La figure 1 illustre selon une vue en coupe longitudinale, le long de la figure A-A de la figure 2, un exemple de réalisation de l'invention, dans un état de non expansion.
- La figure 2 illustre selon une vue de face, un exemple de réalisation de l'invention, dans l'état illustré à la figure 1.
- Les figures 3 et 4 sont des vues similaires aux figures 1 et 2, la figure 3 étant une vue en coupe longitudinale le long de la ligne B-B de la figure 4, les figures 3 et 4 illustrant un exemple de réalisation de l'invention dans un état d'expansion complet.
- Les figures 5 et 6 illustrent selon des vues en perspective respectivement proximale et distale, un exemple de réalisation de l'invention tel que montré aux figures 1 et 2.
- La figure 7 illustre selon une vue en perspective schématique l'implantation d'un dispositif selon l'invention dans le sinus tarsien.

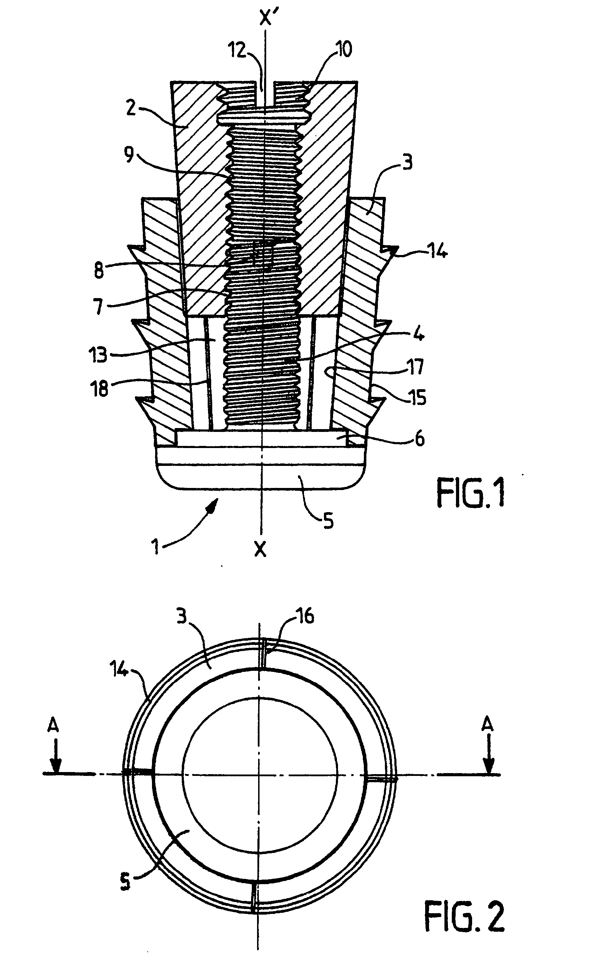

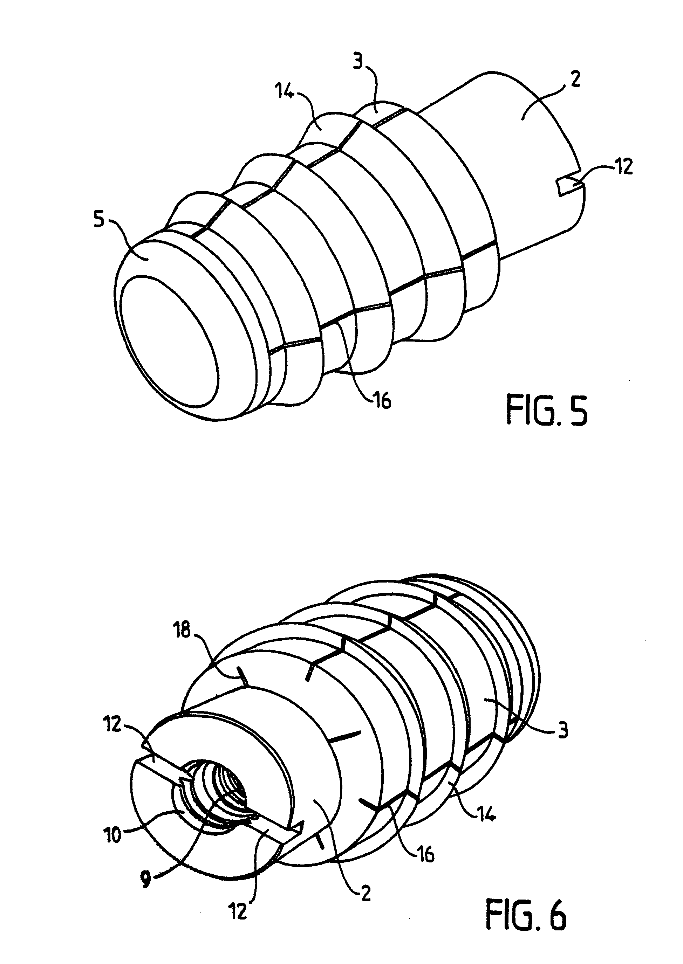

Sur les figures 1 à 6, on a représenté un exemple de réalisation préférentiel de l'invention. Sur les figures 1 et 2, on a représenté un dispositif chirurgical pour pied plat selon la présente invention, avant sa fixation dans la cavité du sinus tarsien. Ce dispositif est constitué de trois éléments : un corps central 1, un cône d'expansion 2 et une couronne 3. Le corps central 1 a la forme générale d'une vis, à savoir comporte une partie cylindrique 4 d'axe longitudinal XX, et possédant une tête 5 au-dessous de laquelle est réalisé un épaulement 6. Le corps central 1 est de préférence métallique, par exemple en titane ou en acier inoxydable. Il a une grande longueur (par exemple de 10 mm à 20 mm) et la tête 5 a pour fonction de bloquer le corps en translation. La mise en place du dispositif selon l'invention se fait par son introduction dans la cavité du sinus tarsien, de manière à ce que la tête 5 vienne en butée contre le fond de la cavité. La tête 5 constitue donc l'extrémité proximale du dispositif, à savoir l'extrémité la plus proche du fond de la cavité.

La partie cylindrique 4 comporte sur toute sa longueur un filetage 7 sur sa face externe. Par exemple, le filetage 7 a un pas constant à gauche.

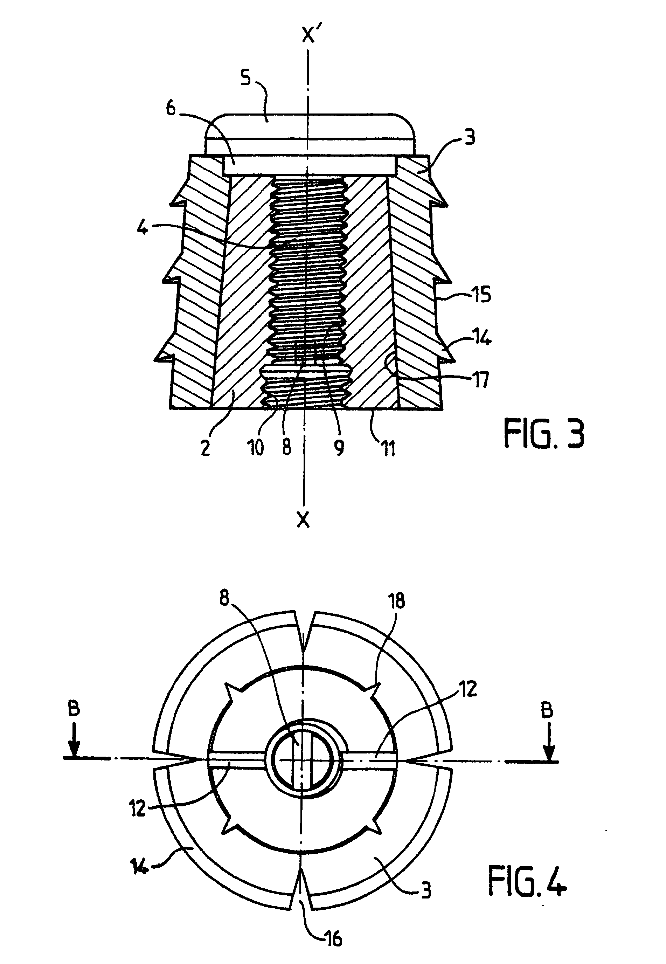

La partie cylindrique 4 comporte à l'extrémité opposée de la tête 5 un usinage 8 qui permet le montage d'un instrument pour l'entraîner en rotation. Cet usinage 8 est de préférence une fente, comme représenté sur la figure 4, dans laquelle peut se loger un tournevis.

Le cône d'expansion 2 est en un matériau déformable ou non, biocompatible et/ou biorésorbable, et peut par exemple être en polyéthylène ou de préférence être métallique.

Le cône d'expansion 2 a la forme générale d'un cône tronqué d'axe longitudinal XX, et a une longueur sensiblement égale à la longueur de la partie cylindrique 4. Son diamètre le plus petit est du côté de l'extrémité distale du dispositif.

Le cône d'expansion 2 est percé de part en part longitudinalement, à savoir il comporte un trou cylindrique 4, aux tolérances près. Il comporte un premier filetage 9 interne du côté de son extrémité proximale, à savoir l'extrémité la plus proche de la tête 5, et un second filetage 10 interne du côté de son extrémité distale, à savoir la plus éloignée de la tête 5. Le premier filetage 9 coopère avec le filetage 7 du corps central 4, et donc est dans le même sens et a le même pas. Par contre, le second filetage 10 est en sens inverse du premier filetage 9. Le premier filetage 9 est réalisé sur environ les trois quarts de la longueur du trou cylindrique, et le second filetage 10 est réalisé sur environ le quart de la longueur du trou cylindrique. Ce second filetage 10 est destiné à recevoir un instrument préhenseur. On peut cependant prévoir un usinage différent d'un filetage pour recevoir un instrument préhenseur.

Du côté du plus grand diamètre, le cône d'expansion 2 comporte en outre une zone sur la face 11 perpendiculaire à l'axe XX longitudinal du dispositif, où est prévu un autre usinage, par exemple une fente 12, pour pouvoir placer un instrument permettant de bloquer en rotation le dispositif selon l'invention.

La face externe du cône d'expansion 2 est totalement lisse, de manière à pouvoir glisser longitudinalement par rapport à la couronne 3. Il en est de même pour la surface interne de la couronne.

La couronne 3 est de forme générale conique et est de préférence en polyéthylène. Elle est percée d'une cavité 13 dans laquelle vient s'emboîter le cône d'expansion 2. La couronne 3 est munie, sur sa face externe 15, d'ailettes 14 qui ont pour fonction de s'opposer à l'expulsion du dispositif selon l'invention, une fois qu'il est en place.

La couronne 3 possède sur sa face externe 15 des entailles 16 qui sont axiales et qui ont une profondeur supérieure à la moitié de l'épaisseur de la couronne 3. De préférence, ces entailles sont au moins au nombre de trois, plus préférentiellement au nombre de quatre, et elles sont prévues pour que leurs bords s'écartent lorsque le cône d'expansion pénètre dans la couronne 3.

Sur la face interne 17 de la couronne 3 sont prévues des entailles 18, qui sont de préférence de même profondeur que les entailles 16 sur la surface externe 15, mais qui sont décalées de 45° par rapport à ces dernières.

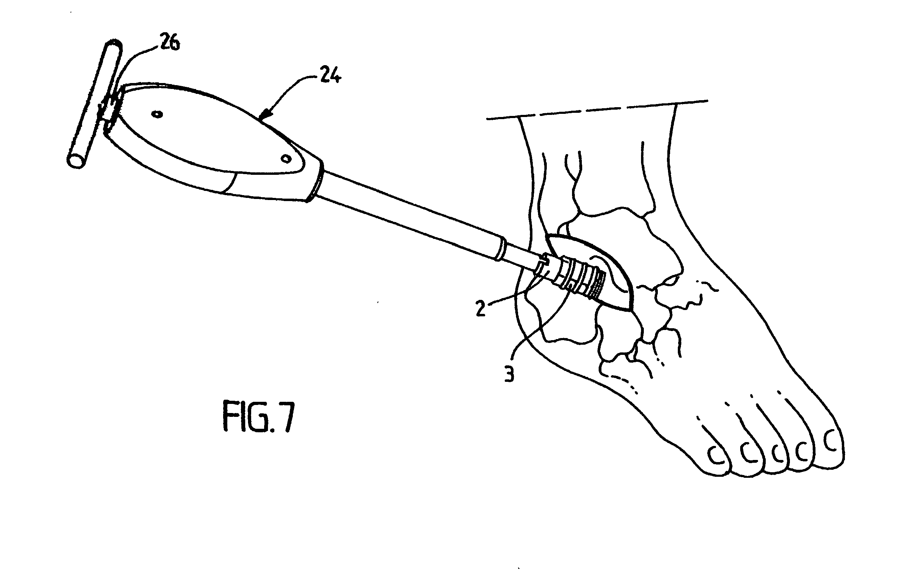

L'insertion du dispositif se fait de la manière suivante (figure 7).

On pratique une seule incision au niveau du canal tarsien et on introduit l'ensemble des trois pièces du dispositif selon l'invention. Le dispositif est introduit au moyen d'un organe spécifique préhenseur (non représenté) qui tient l'extrémité du cône d'expansion 2 par le filetage 10.

Puis, après avoir retiré le préhenseur, on obtient l'expansion du dispositif et donc son blocage dans le canal tarsien, en utilisant un autre organe spécifique, à savoir un premier tournevis 24 qui pénètre dans la fente 12 et qui bloque en rotation le cône d'expansion 2. Ce tournevis est creux et permet le passage en son centre d'un second tournevis 26 de diamètre plus petit, qui pénètre dans la fente 8 et qui permet de faire tourner le corps central 1. Grâce aux filetages 7 et 9, le cône d'expansion 2 pénètre petit à petit dans la couronne 3 et, du fait de la différence de diamètre, pousse radialement la couronne 3.

Comme on le voit plus particulièrement sur les figures 3 et 4, lors du déplacement du cône d'expansion 2, les entailles 16 et 18 s'ouvrent et les ailettes 14 viennent se bloquer contre la cavité du canal tarsien, bloquant ainsi le dispositif dans la cavité.

Le dispositif selon l'invention est remarquable, car il ne nécessite qu'une seule incision et sa géométrie lui permet d'obtenir la meilleure congruence possible dans la cavité du canal tarsien.

## Revendications

1. - Dispositif chirurgical pour pied plat, **caractérisé en ce qu'**il comprend les trois parties suivantes:
- un corps central cylindrique (1) comportant un filetage (7) sur sa partie externe, comportant une tête de blocage (5) en translation,
- un cône d'expansion (2) comportant un filetage (9) interne à sa partie proximale coopérant avec le filetage (7) du corps central (1),
- une couronne externe (3) pouvant être expansée par le cône d'expansion (2) lors du vissage du corps central (1), qui déplace longitudinalement le cône d'expansion (2) dans la couronne externe (3), depuis la partie distale vers la partie proximale.

2. - Dispositif chirurgical selon la revendication 1, **caractérisé en ce que** le cône d'expansion (2), lors du vissage du corps central (1), expanse la couronne externe (3) d'une manière homogène diamétralement.

3. - Dispositif selon l'une des revendications 1 ou 2, **caractérisé en ce que** la couronne externe (3) possède des entailles (16) sur sa surface externe (15) et des entailles (18) sur sa surface interne (17), d'une profondeur supérieure à la moitié de l'épaisseur de la couronne, et décalées angulairement, de manière à ce que ladite couronne externe (3) soit expansée en augmentant son diamètre.

4. - Dispositif selon l'une des revendications précédentes, **caractérisé en ce que** la couronne externe (3) possède des moyens anti-recul, notamment des ailettes (14), dont l'angle médian a une valeur comprise entre 80° et 20° par rapport à l'axe longitudinal du dispositif, de préférence de 45°, et ont une largeur comprise entre 1 mm et 2 mm, de manière à permettre un meilleur blocage du dispositif dans la cavité du sinus tarsien.

5. - Dispositif selon l'une des revendications précédentes, **caractérisé en ce que** le cône d'expansion (2) a un premier filetage (9) dans sa partie proximale, et un second filetage (10) dans sa partie du côté de sa partie distale, mais en sens inverse du premier filetage, de manière à permettre le montage d'un préhenseur.

6. - Dispositif selon l'une des revendications précédentes, **caractérisé en ce que** le corps central (1) possède à sa partie distale un usinage (8), notamment une fente, permettant le montage d'un instrument pour l'entraîner en rotation.

7. - Dispositif selon l'une des revendications précédentes, **caractérisé en ce que** le cône d'expansion (2) a une face externe lisse.

8. - Dispositif selon l'une des revendications précédentes, **caractérisé en ce que** le cône d'expansion (2) comporte un second usinage, notamment une fente (12), pour permettre sa translation le long de la couronne externe (3) pendant la rotation du corps central (1).

9. - Dispositif selon l'une des revendications précédentes, **caractérisé en ce que** la couronne externe est constituée d'un matériau déformable biocompatible et/ou biodégradable, par exemple du polyéthylène, ou un métal.

10. - Dispositif selon l'une des revendications précédentes, **caractérisé en ce que** le corps central et le cône d'expansion sont métalliques, par exemple en titane ou en acier inoxydable.

## Claims

1. Surgical device for flat feet, **characterised in that** it comprises the following three parts:
- a cylindrical central body (1) having a thread (7) on its external part, comprising a head (5) for locking in translation,
- an expansion cone (2) having an internal thread (9) at its proximal part cooperating with the thread (7) of the central body (1),
- an external ring (3) able to be expanded by the expansion cone (2) during the screwing of the central body (1), which moves the expansion cone (2) longitudinally in the external ring (3), from the distal part towards the proximal part.

2. Surgical device according to claim 1, **characterised in that** the expansion cone (2), during the screwing of the central body (1), expands the external ring (3) in a diametrically even manner.

3. Device according to one of claims 1 or 2, **characterised in that** the external ring (3) has notches (16) on its external surface (15) and notches (18) on its internal surface (17), to a depth greater than half the thickness of the ring, and offset angularly, so that the said external ring (3) is expanded by increasing its diameter.

4. Device according to one of the preceding claims, **characterised in that** the external ring (3) has anti-retraction means, in particular fins (14), the median angle of which has a value of between 80° and 20° with respect to the longitudinal axis of the device, preferably 45°, and which have a width of between 1 mm and 2mm, so as to allow better locking of the device in the cavity of the tarsal sinus.

5. Device according to one of the preceding claims, **characterised in that** the expansion cone (2) has a first thread (9) in its proximal part and a second thread (10) in its part on the same side as its distal part, but in the opposite direction to the first thread, so as to allow mounting of a gripper.

6. Device according to one of the preceding claims, **characterised in that** the central body (1) has at its distal part a machining (8), in particular a slot, allowing the mounting of an instrument for driving it in rotation.

7. Device according to one of the preceding claims, **characterised in that** the expansion cone (2) has a smooth external face.

8. Device according to one of the preceding claims, **characterised in that** the expansion cone (2) comprises a second machining, in particular a slot (12), for allowing its translation along the external ring (3) during the rotation of the central body (1).

9. Device according to one of the preceding claims, **characterised in that** the external ring consists of a biocompatible and/or biodegradable deformable material, for example polyethylene, or a metal.

10. Device according to one of the preceding claims, **characterised in that** the central body and the expansion cone are metallic, for example made from titanium or stainless steel.

## Patentansprüche

1. - Chirurgische Vorrichtung für Plattfüße, **dadurch gekennzeichnet, dass** sie die drei folgenden Teile umfasst:
- einen zylindrischen Zentralkörper (1), umfassend ein Gewinde (7) auf seinem äußeren Part, umfassend einen Sperrkopf (5) der Verschiebung,
- einen Ausdehnungskegel (2), umfassend ein inneres Gewinde (9) an seinem proximalen Part, das mit dem Gewinde (7) des Zentralkörpers (1) zusammenwirkt,
- einen äußeren Kranz (3), der durch den Ausdehnungskegel (2) bei der Verschraubung des Zentralkörpers (1), der den Ausdehnungskegel (2) im äußeren Kranz (3) in Längsrichtung verschiebt, vom distalen Teil zum proximalen Teil ausgedehnt werden kann.

2. - Chirurgische Vorrichtung nach Anspruch 1, **dadurch gekennzeichnet, dass** der Ausdehnungskegel (2) bei der Verschraubung des Zentralkörpers (1) den äußeren Kranz (3) auf diametral homogene Weise ausdehnt.

3. - Vorrichtung nach einem der Ansprüche 1 oder 2, **dadurch gekennzeichnet, dass** der äußere Kranz (3) Einkerbungen (16) auf seiner Außenfläche (15) und Einkerbungen (18) auf seiner Innenfläche (17) mit einer Tiefe umfasst, die größer als die Hälfte der Dicke des Kranzes ist, und die winklig verschoben sind, so dass der äußere Kranz (3) durch Erhöhung seines Durchmessers ausgedehnt wird.

4. - Vorrichtung nach einem der vorhergehenden Ansprüche, **dadurch gekennzeichnet, dass** der äußere Kranz (3) Mittel zur Verhinderung des Rücklaufes umfasst, insbesondere Flügel (14), deren mittlerer Winkel einen Wert zwischen 80° und 20° bezogen auf die Längsachse der Vorrichtung aufweist, vorzugsweise von 45°, und die eine Länge aufweisen, die zwischen 1 mm und 2 mm liegt, um eine bessere Blockierung der Vorrichtung im Hohlraum der Tarsalhöhle zu ermöglichen.

5. - Vorrichtung nach einem der vorhergehenden Ansprüche, **dadurch gekennzeichnet, dass** der Ausdehnungskegel (2) ein erstes Gewinde (9) in seinem proximalen Teil und ein zweites Gewinde (10) in seinem Teil an der Seite seines distalen Teils aufweist, jedoch in Gegenrichtung zum ersten Gewinde, um die Montage eines Greifers zu ermöglichen.

6. - Vorrichtung nach einem der vorhergehenden Ansprüche, **dadurch gekennzeichnet, dass** der Zentralkörper (1) an seinem distalen Teil eine Bearbeitung (8) aufweist, insbesondere einen Spalt, der die Montage eines Instrumentes ermöglicht, um ihn zum Drehen zu bringen.

7. - Vorrichtung nach einem der vorhergehenden Ansprüche, **dadurch gekennzeichnet, dass** der Ausdehnungskegel (2) eine glatte Außenseite aufweist.

8. - Vorrichtung nach einem der vorhergehenden Ansprüche, **dadurch gekennzeichnet, dass** der Ausdehnungskegel (2) eine zweite Bearbeitung umfasst, insbesondere einen Spalt (12), um seine Verschiebung entlang der äußeren Kranzes (3) während der Drehung des Zentralkörpers (1) zu ermöglichen.

9. - Vorrichtung nach einem der vorhergehenden Ansprüche, **dadurch gekennzeichnet, dass** der äußere Kranz aus einem verformbaren biokompatiblen und/oder biologisch abbaubaren Werkstoff hergestellt ist, z.B. aus Polyäthylen, oder einem Metall.

10. - Vorrichtung nach einem der vorhergehenden Ansprüche, **dadurch gekennzeichnet, dass** der Zentralkörper und der Ausdehnungskegel aus Metall sind, z.B. aus Titan oder aus Edelstahl.
